# EUROPEAN PATENT SPECIFICATION

(11) **EP 3 517 620 B1**
(45) Date of publication and mention of the grant of the patent: **22.12.2021**
(21) Application number: 17853075.4
(22) Date of filing: 20.09.2017
(51) Int. Cl.: C12P 13/06, C12P 13/04, C12P 13/12, C12N 9/04, C12N 9/10, C12N 15/09, C12N 15/66

(54) **METHOD FOR PRODUCING L-CYSTEINE**
VERFAHREN ZUR HERSTELLUNG VON L-CYSTEIN
PROCÉDÉ DE PRODUCTION DE L-CYSTÉINE

(30) Priority: 21.09.2016 JP 2016184743
(43) Date of publication of application: 31.07.2019
(73) Proprietor: KOHJIN Life Sciences Co., Ltd., Tokyo 100-0006 (JP); Osaka University, Suita-shi, Osaka 565-0871 (JP)
(72) Inventor: HONDA, Kohsuke, Suita-shi Osaka 565-0871 (JP); IMURA, Makoto, Saiki-shi Oita 876-8580 (JP); IWAKIRI, Ryo, Saiki-shi Oita 876-8580 (JP)
(74) Representative: Müller-Boré & Partner Patentanwälte PartG mbB
(86) International application number: PCT/JP2017/033901
(87) International publication number: WO 2018/056305

(56) References cited:
- EP-A1- 2 444 481
- WO-A1-2010/034672
- JP-A- S6 460 383
- JP-A- 2013 543 723
- SHIMIZU ET AL: "Molecular and functional characterization of d-3-phosphoglycerate dehydrogenase in the serine biosynthetic pathway of the hyperthermophilic archaeon Sulfolobus tokodaii", ARCHIVES OF BIOCHEMISTRY AND BIOPHYSICS, ACADEMIC PRESS, US, vol. 470, no. 2, 22 November 2007 (2007-11-22), pages 120-128, XP022442491, ISSN: 0003-9861, DOI: 10.1016/J.ABB.2007.11.010

## Description

### Technical Field

The present invention relates to a method for producing O-phosphoserine and the amino acid L-cysteine, using the combination of enzymes derived from a thermophilic bacterial 3-phosphoglycerate dehydrogenase (PGDH) and phosphoserine aminotransferase (PSAT).

### Background Art

L-cysteine is an amino acid that has been rapidly expanding the market in recent years, and as a result of the increase in the world population, it is expected that the market will continue to expand. The main use of L-cysteine is the use as a precursor for the synthesis of food additives, pharmaceuticals, cosmetics, glutathione, N-acetyl-cysteine, or coenzyme A.

As a method for producing L-cysteine, separation and extraction from an acid hydrolysate of hairs, feathers, or the like are mainly performed, but in recent years, production by fermentation method by microorganisms has also been started. However, the fermentation method by microorganisms has the following problems. It is known that as the intracellular concentration increases, L-cysteine causes (1) growth inhibition of microorganisms, and (2) feedback inhibition to biosynthetic enzymes. With respect to (1), the problem has been solved by identification of an L-cysteine extracellular efflux pump protein and enhancement of expression of the gene, and with respect to (2), the problem has been solved by breeding strategy of conformational analysis of an L-cysteine biosynthetic enzyme and elucidation of a feedback inhibition mechanism, and creation of an insensitive mutant enzyme based on the analysis and the elucidation. As described above, attempts have been made to improve the productivity by genetic modification, breeding, or the like, by utilizing the metabolic function of microorganisms, however, due to the above-described problems, sufficient productivity has not been obtained in some cases (Patent Literatures 1 to 5).

Further, by using an enzyme derived from microorganisms, various kinds of useful substances are produced (Patent Literatures 6 and 7). When L-cysteine is produced by such a technique, O-phosphoserine may be synthesized in the middle of the production. This O-phosphoserine is synthesized from 3-phosphoglyceric acid (hereinafter, referred to as "3PG" in principle) via phosphohydroxypyruvic acid (hereinafter, referred to as "HPV" in principle). In Non Patent Literature 1, 3-phosphoglycerate dehydrogenase (hereinafter, referred to as "PGDH" in principle) derived from Sulfolobus tokodaii that is hyperthermophile archaea has been reported, however, the enzymatic activity can be confirmed only in PGDH overexpressed in E. coli that is designed to promote disulfide bonds under extremely extreme conditions such as pH 11, and the activity has not been substantially observed at pH 8.0 that is a value in the vicinity of the neutrality where the bacterial cells can survive originally. The inventors of the present application have measured the enzymatic activity by cloning PGDH derived from Thermococcus kodakarensis KOD1 that has already been annotated, however, similarly the enzymatic activity has not been found by PGDH alone.

Under these circumstances, in order to address a growing demand in the future, development of an inexpensive and efficient production method of L-cysteine is required.

### Citation List

### Patent Literature

Patent Literature 1: JP 2010-22215 A
Patent Literature 2: WO 2013/000864
Patent Literature 3: JP 2009-232844 A
Patent Literature 4: Re-publication of PCT International Publication No. 2012-137689
Patent Literature 5: WO 2012/152664
Patent Literature 6: JP 2005-160371 A
Patent Literature 7: JP 2003-219892 A

### Non Patent Literature

Non Patent Literature 1: 1: Shimizu Y, Sakuraba H, Doi K, Ohshima T (2008) Molecular and functional characterization of D-3-phosphoglycerate dehydrogenase in the serine biosynthetic pathway of the hyperthermophilic archaeon Sulfolobus tokodaii. Arch Biochem Biophys. 470(2): 120-8.

### Summary of Invention

### Technical Problem

An object of the present invention is to provide a novel method for producing L-cysteine in place of a conventional fermentation method. More specifically, the object is to provide a method for producing L-cysteine by the combination of heat-resistant enzymes. In particular, the object is to provide a method for efficiently producing a pathway for synthesizing O-phosphoserine from 3PG via HPV.

### Solution to Problem

As a result of the intensive studies to solve the problems described above, the present inventors have found a method for producing L-cysteine by a technique of combining heat-resistant enzymes and constructing an arbitrary artificial metabolic pathway in vitro. The method of the invention was done as follows:
(1) a heat-resistant enzyme gene derived from a thermophilic/hyperthermophilic bacterium is expressed in a general-purpose and mesophilic microbial host such as E. coli;
(2) the obtained recombinant bacterial cells or an extract from bacterial cells is subjected to a heat treatment at around 60 to 90°C to inactivate an enzyme derived from a host and further to partially destroy a cell structure of the host, and a catalytic module with enhanced permeability of a substrate/product is obtained; and
(3) the modules are combined to construct an in vitro artificial metabolic pathway.

In addition, as for the synthesis of O-phosphoserine, an activity has been found by the combination of PGDH and phosphoserine aminotransferase (hereinafter, referred to as "PSAT" in principle), which are each derived from different species of a bacterium. Due to the synthesis of O-phosphoserine, it has been revealed that PGDH and PSAT each by itself do not function, however, an activity is exhibited by the combination of the PGDH and the PSAT.

In this way, an object of the present invention is to provide a novel method for producing L-cysteine inplace of a conventional fermentation method. More specifically, the object is to provide a method for producing L-cysteine by the combination of heat-resistant enzymes. In particular, a method for producing O-phosphoserine that is a precursor of L-cysteine from 3PG via HPV has been found.

More specifically, the present invention provides the following inventions:
(1) a method for producing O-phosphoserine, including acting 3-phosphoglycerate dehydrogenase (PGDH) and phosphoserine aminotransferase (PSAT) that are each derived from a thermophilic bacterium on 3-phosphoglyceric acid (3PG) at pH of 6 to 8 to generate O-phosphoserine;
(2) a method for producing L-cysteine, including the production step of (1); and
(3) the method for producing L-cysteine of (2), further including adding glutamate dehydrogenase (GDH) or NADH oxidase (hereinafter, also referred to as "NOX").

### Advantageous Effects of Invention

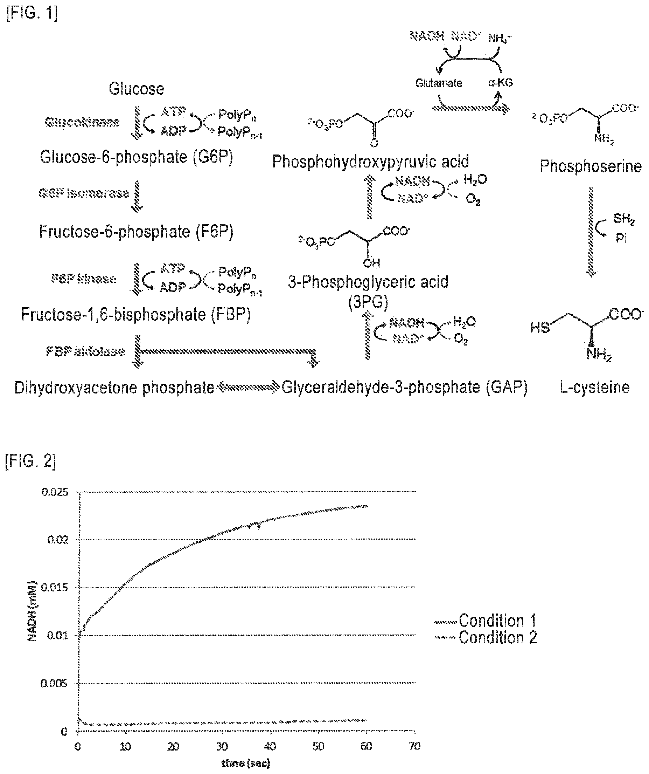

According to the present invention, L-cysteine can be produced in vitro by using a heat-resistant enzyme obtained from microorganisms by roughly purifying the microorganisms. Therefore, growth inhibition by L-cysteine, which has been a problem in a fermentation method, does not become problematic. Further, in the present invention, by the combination of enzymes, a pathway can be freely designed, and therefore, the design can be made so as not to go through an enzyme reaction that give feedback inhibition by L-cysteine (Fig. 1).

### Brief Description of Drawings

Fig. 1 shows an example of a metabolic pathway of L-cysteine production.
Fig. 2 shows activity measurement of PGDH.
Fig. 3 shows results of activity measurement of PSAT.
Fig. 4 shows concentration of each of NADH and α-KG in a reaction solution.
Fig. 5 shows activity of PGDH.
Fig. 6 shows investigation of addition of a coenzyme regeneration system.
Fig. 7 shows time-dependent change of a cysteine production test.

### Description of Embodiments

The present invention is a method for producing O-phosphoserine, including acting phosphoserine aminotransferase (PSAT) and 3-phosphoglycerate dehydrogenase (PGDH) that are each derived from a thermophilic bacterium on 3-phosphoglyceric acid (3PG) to generate O-phosphoserine. Further, the present invention is a method for producing L-cysteine, including the step described above.

O-phosphoserine is synthesized from 3PG via HPV. The enzymes to be used in this pathway are PGDH and PSAT.

The 3-phosphoglycerate dehydrogenase (PGDH) that is used in the present invention is an oxidoreductase that converts 3PG to HPV. The structure and the like of the enzyme are not particularly limited as long as the enzyme is derived from a thermophilic bacterium. In the present invention, the thermophilic bacterium is a bacterium that expresses an enzyme capable of maintaining the activity without denaturation at 60°C or more, and particularly 70 to 110°C. Examples of the thermophilic bacterium include the genus Thermus (suchasThermus thermophilus, and Thermus aquaticus), the genus Thermotoga (such as Thermotoga lettingae, Thermotoga neapolitana, Thermotoga petrophila, and Thermotoga maritima), the genus Thermococcus (such as Thermococcus profundus, Thermococcus kodakarensis, and Thermococcus gammatolerans), the genus Pyrococcus (such as Pyrococcus horikoshii, Pyrococcus abyssi, Pyrococcus glycovorans, Pyrococcus furiosus, and Pyrococcus wosei), the genus Sulfolobus (such as Sulfolobus tokodaii, Sulfolobus acidocaldarius, Sulfolobus islandicus, and Sulfolobus solfataricus), the genus Pyrodictium (such as Pyrodictium occultum, Pyrodictium abyssi, and Pyrodictium brockii), the genus Pyrobaculum (such as Pyrobaculum aerophilum, Pyrobaculum arsenaticum, and Pyrobaculum organotrophum), the genus Hyperthermus (such as Hyperthermus butylicus), the genus Aquifex (such as Aquifex pyrophilus) , the genus Thermodesulfobacterium (such as Thermodesulfobacterium commune), the genus Methanopyrus (such as Methanopyrus kandleri), the genus Pyrolobus (such as Pyrolobus fumarii), the genus Thermofilum (such as Thermoproteus tenax, and Thermoproteus naphthophila), the genus Thermosynechococcus (such as Thermosynechococcus elongates), the genus Synechococcus (such as Synechococcus lividus), the genus Oceanithermus (such as Oceanithermus profundus), the genus Rhodothermus (such as Rhodothermus marinus), the genus Thermovibrio (such as Thermovibrio ammonificans), the genus Desulfurobacterium (such as Desulfurobacterium thermolithotrophum), and the genus Thermodesulfatator (such as Thermodesulfatator indicus).

Next, phosphoserine aminotransferase (PSAT) is a serine biosynthetic enzyme. The structure and the like of the enzyme are not particularly limited as long as the enzyme is derived from a thermophilic bacterium. In the present invention, the thermophilic bacterium is a bacterium that expresses an enzyme capable of maintaining the activity without denaturation at 60°C or more, and particularly 70 to 110°C. Examples of the thermophilic bacterium include the genus Thermus (such as Thermus thermophilus, and Thermus aquaticus), the genus Thermotoga (such as Thermotoga lettingae, Thermotoga neapolitana, Thermotoga petrophila, and Thermotoga maritima), the genus Thermococcus (such as Thermococcus profundus, Thermococcus kodakarensis, and Thermococcus gammatolerans), the genus Pyrococcus (such as Pyrococcus horikoshii, Pyrococcus abyssi, Pyrococcus glycovorans, Pyrococcus furiosus, and Pyrococcus wosei), the genus Sulfolobus (such as Sulfolobus tokodaii, Sulfolobus acidocaldarius, Sulfolobus islandicus, and Sulfolobus solfataricus), the genus Pyrodictium (such as Pyrodictium occultum, Pyrodictium abyssi, and Pyrodictium brockii), the genus Pyrobaculum (such as Pyrobaculum aerophilum, Pyrobaculum arsenaticum, and Pyrobaculum organotrophum), the genus Hyperthermus (such as Hyperthermus butylicus), the genus Aquifex (such as Aquifex pyrophilus), the genus Thermodesulfobacterium (such as Thermodesulfobacterium commune), the genus Methanopyrus (such as Methanopyrus kandleri), the genus Pyrolobus (such as Pyrolobus fumarii), the genus Thermofilum (such as Thermoproteus tenax, and Thermoproteus naphthophila), the genus Thermosynechococcus (such as Thermosynechococcus elongates), the genus Synechococcus (such as Synechococcus lividus), the genus Oceanithermus (such as Oceanithermus profundus), the genus Rhodothermus (such as Rhodothermus marinus), the genus Thermovibrio (such as Thermovibrio ammonificans), the genus Desulfurobacterium (such as Desulfurobacterium thermolithotrophum), and the genus Thermodesulfatator (such as Thermodesulfatator indicus).

There are no particular restrictions on the method for acquiring each of the PGDH and the PSAT as long as the enzymes to be used in the present invention are each derived from a thermophilic bacterium such as S. tokodaii or the like as described above. In a case where the enzymes are obtained by using a technique of genetic engineering, the gene encoding a desired enzyme is inserted into an appropriate vector to construct a recombinant vector. The recombinant vector is transformed into a host cell capable of producing an enzyme, and an enzyme can be expressed and produced. In the present invention, since multiple enzymes are used, E. coli such as DH5α and MG1655 strains, a Gram-negative bacterium such as the genus Pseudomonas, a Gram-positive bacterium such as the genus Corynebacterium, the genus Bacillus, and the genus Rhodococcus, which are easily transformable, are suitable. Specifically, the PGDH and the PSAT are each expressed under the control of a T7 promoter by ligating the gene obtained by PCR amplification of agenomic DNA of each ofmicroorganisms, forexample, topET21a. The genomic DNA of each of microorganisms can be obtained from National Research and Development Institute of RikenBioResource Research Center; National Institute for Environmental Studies; Biological Resource Center (NBRC), Incorporated Administrative Agency National Institute of Technology and Evaluation; Public Interest Incorporated Foundation Kazusa DNA Research Institute; or the like. Further, as the DNA to be used, a DNA synthesized from sequence information can also be used, and the DNA may not be completely identical with the sequence information as long as it has the same amino acid sequence. The synthetic DNA is a DNA sequence designed by matching the amino acid sequence to the codon usage frequency of E. coli. An expression vector in which the gene is prepared from such a DNA as described above is transformed into an E. coli such as BL21 (DE3) pLysS manufactured by Novagen. Alternatively, a DNA fragment by homologous recombination or transposon may be inserted. As the transformation method, a general method may be used.

In the present invention, the enzymes to be used in the production of O-phosphoserine from 3PG are PSAT and PGDH. The enzymes each derived from a thermophilic bacterium described up to the preceding stage can be appropriately selected and used. These two enzymes may be derived from the same species of a bacterium or derived from different species of a bacterium. In a production step of O-phosphoserine, O-phosphoserine can be obtained in the vicinity of the neutrality of around pH 6 to 8 by using these two enzymes even in a case where the PGDH by itself reacts only under the conditions of pH 11 or the like.

As the enzymes required in the invention of the present application, multiple enzymes can be expressed and obtained simultaneously in bacterial cells selected from E. coli such as DH5α and MG1655 strains, a Gram-negative bacterium such as the genus Pseudomonas, a Gram-positive bacterium such as the genus Corynebacterium, the genus Bacillus, and the genus Rhodococcus. The bacterial cells used in this paragraph are not required to be thermophilic bacteria in order to easily remove a protein derived from a host as described later. All of the required enzymes may be expressed at the same time, but usually, because of the expression efficiency or the like, the enzymes may be obtained by dividing the bacterial cells into multiple types of E. coli and the like, as described in Examples. Further, each of the enzymes may be expressed in individual E. coli or the like. In the present application, since the enzymes each derived from a thermophilic bacterium are obtained by the expression in E. coli or the like, a protein derived from a host such as E. coli or the like can be easily removed. For example, after expression in E. coli, by performing heat treatment at a high temperature of 60°C to 80°C, a protein derived from a host is denatured, however, the desired enzymes are enzymes each derived from a thermophilic bacterium, and therefore, are not heat-denatured. In this way, by removing the proteins denatured by heat treatment, the required crude enzyme solution can be easily obtained. In the heat treatment, the bacterial cells after cultivation may be directly heat-treated. Alternatively, the bacterial cell extract may be heat-treated. The extraction method can be selected without any particular limitation. After disrupting the bacterial cells by sonication or the like, the extracted liquid may be heat-treated. Specifically, for example, the wet cells of the recombinant E. coli are suspended in 50 mM HEPES-NaOH (pH 8.0) so as to be 200 mg wet cells/ml, and the suspension is subjected to sonication to disrupt the bacterial cells, and a cell-free extract is obtained. The cell-free extract is heat-treated at 70°C for 30 minutes to denature the host-derived proteins. By removing the cell residues and the denatured proteins by centrifugation, the supernatant can be used as a crude enzyme solution for the production of L-cysteine.

In the present invention, among the steps of producing L-cysteine, a step of obtaining O-phosphoserine includes a step of obtaining O-phosphoserine from 3PG by the PGDH and PSAT described above. Since the PGDH and PSAT each by itself do not function, both of the enzymes PGDH and PSAT use at the same time. As the enzymes, the above-described enzymes each derived from a thermophilic bacterium are used, and the enzymes may be each derived from the same species of a thermophilic bacterium or derived from different species of a thermophilic bacterium, and can be used in appropriate combination.

As also an enzyme required in a step other than the step of obtaining O-phosphoserine from 3PG of the preceding stage among the steps of producing the L-cysteine of the present invention, an enzyme derived from a thermophilic bacterium is used similarly as in PGDH and PSAT. Any enzyme can be appropriately selected and used as long as it is derived from a thermophilic bacterium. The enzyme to be used differs depending on the raw material, and an example of the enzyme group required in a case where glucose is used as a raw material is shown in Table 1. Other raw materials are a saccharide such as glycerol, and starch, and any raw materials can be accepted as long as they can be metabolized by the glycolytic pathway. In the present application, if there is a step of obtaining O-phosphoserine by PGDH and PSAT, the raw materials described above can be used without any limitation. An enzyme when a raw material other than glucose is used can be appropriately selected by a person skilled in the art. As the required enzymes, enzymes each derived from a thermophilic bacterium, which are mentioned in the present specification, are used.

**[Table 1]**

| | Enzyme name | EC number | Abbreviation |
|---|---|---|---|
| 1 | Glucokinase | 27.1.2 | GK |
| 2 | Glucose phosphate isomerase | 53.1.9 | GPI |
| 3 | Phosphofructokinase | 27.1.1 | PFK |
| 4 | Fructose-1,6-bisphosphate aldolase | 4.1.2.13 | FBA |
| 5 | Triose phosphate isomerase | 53.1.1 | TIM |
| 6 | Glyceraldehyde-3-phosphate dehydrogenase | 1.2.1.90 | GAPN |
| 7 | 3-phosphoglycerate dehydrogenase | 1.1.1.95 | PGDH |
| 8 | Phosphoserine aminotransferase | 2.6.1.52 | PSAT |
| 9 | O-phosphoserine sulfhydrylase | 2.5.1.65 | CysS |
| 10 | Polyphosphate kinase | 27.4.1 | PPK |
| 11 | NADH oxidase | 16.3.4 | NOX |
| 12 | Glutamate dehydrogenase | 14.1.3 | GDH |

The method for acquiring each of the enzymes in Table 1 is performed in a similar manner as in the method described in paragraphs [0017] to [0018] . All of the enzymes may be the same species of a thermophilic bacterium or may be different species of a thermophilic bacterium. As the enzyme gene, a gene available from National Research and Development Institute of Riken BioResource Research Center; National Institute for Environmental Studies; Biological Resource Center (NBRC), Incorporated Administrative Agency National Institute of Technology and Evaluation; Public Interest Incorporated Foundation Kazusa DNA Research Institute; or the like can be used. From the obtained DNA, a target gene can be amplified by PCR or the like and used. Further, a DNA synthesized from the obtained gene information may be used. In the present invention, as described above, in order to introduce a gene into E. coli or the like and express the gene, each enzyme gene may be optimized for expression in a selected cell such as E. coli or the like and used.

The gene thus obtained can be incorporated into an E. coli expression vector by a general method. Although all of the genes to be used can be incorporated into one expression vector, the genes may be introduced separately into expression vectors required depending on the optimality of the microorganism-derived genes to be used, the promoter selection, and the like. For example, fructose-1, 6-bisphosphate aldolase derived from Thermus thermophilus HB8 (TtFBA), glyceraldehyde-3-phosphate dehydrogenase derived from Thermoproteus tenax (TteGAPN), glutamate dehydrogenase derived from Thermoproteus tenax (TteGDH), 3-phosphoglycerate dehydrogenase derived from Thermococcus kodakarensis KOD1 (TkPGDH), and phosphoserine aminotransferase derived from Thermococcus kodakarensis KOD1 (TkPSAT) ligate the gene obtained by PCR amplification of a genomic DNA of each of microorganisms to pET21a, and O-phosphoserine sulfhydrylase derived from Aeropyrum pernix (AmCysS), and NADH oxidase derived from Thermococcus profundus (TpNOX) ligate a synthetic DNA to pET21a, lactate dehydrogenase derived from T. thermophilus HB8 introduces a synthetic DNA to pET11a by a T7 promoter, glucokinase derived from T. thermophilus HB8 (TtGK), glucose phosphate isomerase derived from T. thermophilus HB8 (TtGPI), phosphofructokinase derived from T. thermophilus HB8 (TtPFK), triose phosphate isomerase derived from T. thermophilus HB8 (TtTIM), polyphosphate kinase derived from T. thermophilus HB27 (TtPPK), and pyruvate kinase derived from T. thermophilus HB27 (TtPK) each introduce a synthetic DNA to pRCI(*). Depending on the gene to be used, multiple most suitable vectors may be selected by a person skilled in the art.
* Ninh PH, Honda K, Sakai T, et al. (2015) Assembly and multiple gene expression of thermophilic enzymes in Escherichia coli for in vitro metabolic engineering. Biotechnol Bioeng 112, 189-196

The expression vector thus obtained is introduced into the E. coli or the like described in paragraph 0019. There are no particular restrictions on the method of introduction, and a general method can be used. The cultivation of E. coli and the like may be performed by a general method. In a case of requiring expression induction by a promoter to be used, the expression is induced.

After the cultivation of E. coli, a crude enzyme solution is obtained in a similar manner as in the method described in paragraph 0019. In a case where the bacterial cells used are different, the bacterial cells may be prepared separately or may be prepared collectively. Since enzymes each derived from a thermophilic bacterium are used, a protein derived from a host such as E. coli or the like can be easily removed due to the heat denaturation by heat treatment. In addition, in the present invention, a crude enzyme solution obtained by a purification operation of only removing the denatured proteins can be used. Further, the obtained crude enzyme solution may be further purified to be used.

The crude enzyme solution thus obtained is used for the production of L-cysteine. A coenzyme and a substrate, which are required for a buffer solution, are added to the crude enzyme solution to produce L-cysteine. The coenzyme and the substrate that are to be added vary depending on the raw material (kind of a saccharide) to be used, and in a case where glucose is used as a raw material, into the crude enzyme solution obtained by the method described above, oxidation-type nicotinamide adenine dinucleotide (NAD⁺), adenosine triphosphate (ATP), glucose 1-phosphate (G1P), glutamic acid, sodium sulfide, ammonium sulfate, polyphosphoric acid are added, and into the resultant mixture, glucose is added to produce L-cysteine. Even in a case where glycerol is usedas a rawmaterial, glycerol is added instead of glucose to produce L-cysteine. In the present invention, the reaction of the production is performed at 50°C to 80°C. As a result, there are advantages of improving the solubility of the raw material, increasing the reaction rate, reducing the risk of contamination, and the like. In a case where starch is used as a raw material, an enzyme required for the starch degradation is added.

For the synthesis of L-cysteine, NAD⁺ that is an oxidation-reduction coenzyme being a coenzyme is used. However, since the reaction reaches the equilibrium due to the accumulation of α-ketoglutarate (hereinafter referred to as "α-KG") and reduced nicotinamide adenine dinucleotide (NADH), the production of L-cysteine may not proceed efficiently. In this regard, by using GDH that converts α-KG to glutamic acid or NADH oxidase (NOX) that oxidizes NADH together with other enzymes, the accumulation of α-KG and NADH are improved, and the production rate of L-cysteine can be improved. With respect to the GDH and the NOX, one GDH or one NOX may be added, and when both of the GDH and the NOX are added, the production rate is further improved, and therefore, this is preferred.

After completion of the enzyme reaction described above, L-cysteine can be obtained with the recovery from the enzyme reaction solution. From this reaction solution, L-cysteine can be isolated and purified by a general method that is used for isolation and purification of amino acids. Specifically, for example, gel chromatography, ion exchange chromatography, affinity chromatography, ammonium sulfate precipitation and the like can be performed alone or in appropriate combination.

### Examples

The present invention will be described in more detail below, but is not limited to the following methods.

### (Investigation of step of O-phosphoserine from 3PG)

### · Preparation of enzyme solution

The wet cells of recombinant E. coli were suspended in 50 mM HEPES-NaOH (pH 8.0) so as to be 200 mg wet cells/ml. The suspension was subjected to sonication to disrupt the bacterial cells, and a cell-free extract was obtained. The cell-free extract was heat-treated at 70°C for 30 minutes to denature the host-derived proteins. The supernatant in which cell residues and denatured proteins had been removed by centrifugation was used for activity measurement as a crude enzyme solution.

### · Used enzyme gene, strain, and cultivation

The PGDH and PSAT derived from S. tokodaii (St) ligated a synthetic DNA to pET21a, and the expression was performed under T7 promoter control. On the other hand, the PGDH and PSAT derived from Thermoproteus tenax (Tt), and Thermotoga maritima (Tm), T. kodakarensis (Tk), or Thermosynechococcus elongates (Te) ligated the gene obtained by PCR amplification of a genomic DNA of each of microorganisms to pET21a, and the expression was performed under T7 promoter control. All of these gene expression vectors were introduced into Rosetta 2 (DE3) pLysS manufactured by Novagen. The Rosetta 2 (DE3) pLysS was aerobically cultured at 37°C in a Luria-Bertani medium in which 100 mg/L of ampicillin and 34 mg/L of chloramphenicol had been added. In the late logarithmic growth phase, 0.2 mM IPTG was added to the culture solution to induce a desired enzyme gene.

### · Confirmation of activity of PGDH and PSAT

A reaction solution was prepared with the composition of Table 2 except for 3PG. The reaction solution was warmed at 70°C for one minute, and then 3PG was added to the warmed reaction solution to initiate a reaction. The reaction was measured by the concentration of NADH generated by PGDH with the monitoring of the absorbance at 340 nm. The results are shown in Fig. 2. Note that the concentration of NADH was calculated with a molar extinction coefficient of 6.3 × 10³ mol⁻¹ L⁻¹ cm⁻¹ at 340 nm. As a result, it was revealed that the reaction of PGDH proceeded by adding PSAT.

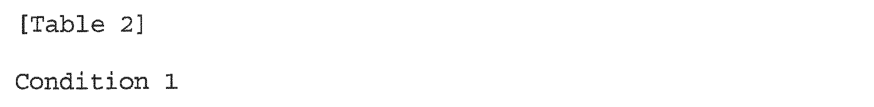

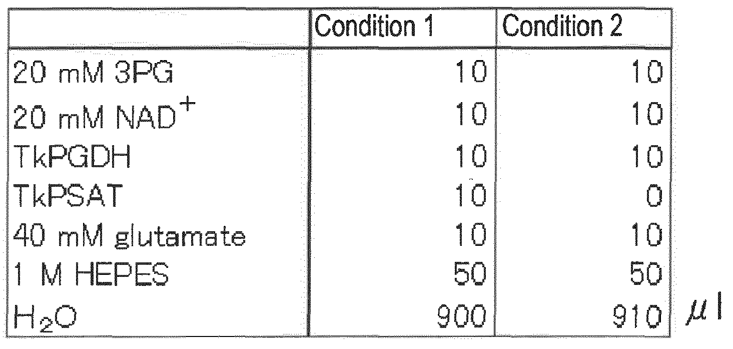

### · Confirmation of activity of PSAT

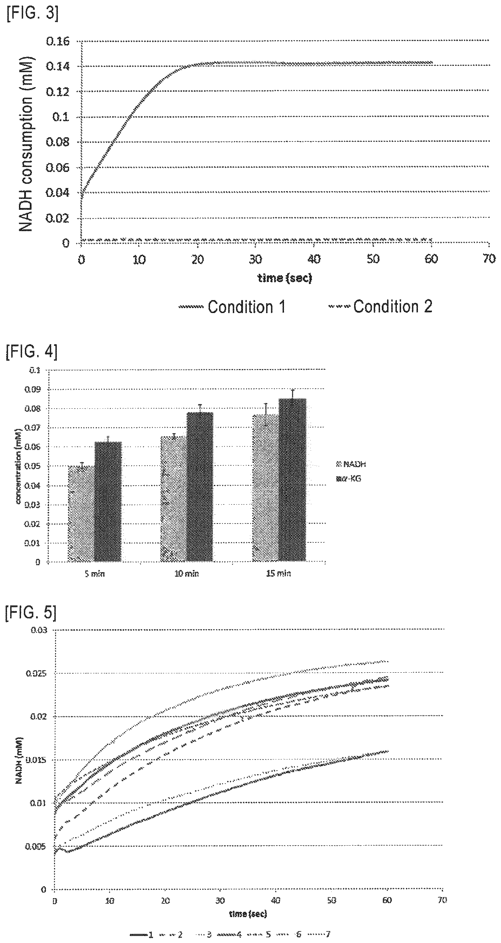

The PGDH enzyme by itself did not exhibit any activity. It was also investigated whether or not PSAT exhibited any activity. However, since the case of the PSAT was not a reaction using NADH, the PSAT by itself was not able to be evaluated. Accordingly, it was investigated whether or not the reaction proceeded by coupling with NAD(H)-dependent glutamate dehydrogenase (TteGDH) . At first, it was confirmed that TteGDH was active under condition 1 of Table 3. Subsequently, it was verified whether or not TkPSAT functioned by coupling TkPSAT and TteGDH under condition 2. The results are shown in Fig. 3, where the consumption of NADH was calculated on the basis of the molar extinction coefficient of 6.3 × 10³ mol⁻¹ L⁻¹ cm⁻¹ at 340 nm. According to this, it was revealed that TkPSAT by itself did not function similarly as in the case of TkPGDH.

**[Table 3]**

| Condition 1 | | | Condition 2 | |
|---|---|---|---|---|
| | *µ*l | | | *µ*l |
| 1 M HEPES (ₚH8.0) | 50 | | 1 M HEPES (ₚH8.0) | 50 |
| 0.1 M a-KG | 10 | | 20 mM NADH | 10 |
| 20 mM NADH | 10 | | 320 mM CH₃COONH₄ | 10 |
| 320 mM OH₃COONH₄ | 100 | | 100 mM glutamate | 20 |
| TteGDH | 1 | | 50 mM HPV | 10 |
| H₂O | 829 | | TteGDH | 10 |
| | | | TkPSAT | 10 |
| | | | H₂O | 790 |

### · Confirmation of PGDH and PSAT

It was revealed that PGDH and PSAT each by itself did not exhibit any enzymatic activity. However, in the above-described technique, only the behavior with NADH was tracked, and therefore, PSAT did not necessarily function in practice. Accordingly, it was investigated whether or not α-KG that is one of products of PSAT was produced. The activity of NADH was measured by using one prepared with the reaction composition shown in Table 4.

With respect to the conditions of an absorption spectrometer, blank measurement was performed at a wavelength of 340 nm and 60°C for 2 minutes, and the values of absorbance at 5, 10 and 15 minutes after the addition of substrate and the α-KG were measured by high-performance liquid chromatography (HPLC) with the partial modification of that of the literature of *2. Specifically, 50 µl was sampled, 40 µl of 2M HCl was added to the sample, and the mixture was centrifuged to obtain a supernatant, into the supernatant, an equal amount of 1 mg/ml o-Phenylenediamine in 3M HCl was added, the resultant mixture was incubated at 80°C for 60 minutes, the incubated mixture was ice-cooled and centrifuged, and HPLC analysis was performed to measure the α-KG. The measurement was performed under the following conditions of: an Eluent of Acetic acid / water / methanol (1:54:45, v/v); a Flow Rate of 0.4 ml/min; a Column of COSMOSIL Packed Column 5C18-AR-II 4.6ID × 250 mm; a Column Temperature of 35°C; a Detection with (Exc = 336 nm and Emi = 420 nm) ; and a Sample cooler of 4°C. The NADH generation amount and the α-KG generation amount are shown in Fig. 4. From these results, the NADH and the α-KG showed similar results. Therefore, it was revealed that the PGDH and the PSAT reacted by linking with each other. *2: Muhling J, Fuchs M, Campos ME, Gonter J, Engel JM, Sablotzki A, Menges T, Weiss S, Dehne MG, Krull M, Hempelmann G. (2003) Quantitative determination of free intracellular α-keto acids in neutrophils. J Chromatogr B 789: 383-392

**[Table 4]**

| | *µ*I |
|---|---|
| 1 M HEPES (ₚH8.0) | 100 |
| 50 m M MnCl₂ | 10 |
| 1 M MnCl₂ | 5 |
| 50 mM NAD⁺ | 20 |
| 1 M glutamate | 20 |
| 20 mM 3PG | 5 |
| PGDH | 0.8 |
| PSAT | 0.8 |
| H₂O | 838.4 |

### · Evaluation of enzymatic activities of various thermophile archaea and bacteria of PGDH and PSAT

From the above, PGDH and PSAT derived from T. kodakarensis functioned when both of the PGDH and the PSAT coexisted. In this regard, it was investigated with the combination of Table 6 whether or not a different thermophile archaeon or a different bacterium also similarly functioned in combination of PGDH and PSAT with the composition of Table 5. Similarly, these enzymes each by itself did not function, but the activity was able to be confirmed by the combination of PGDH and PSAT (Fig. 5). In addition, it was revealed that also by the combination of PGDH and PSAT of 6 and 7 in Table 6, the PGDH and PSAT were each derived from different species of a bacterium, the activity was exhibited.

**[Table 5]**

| | *µ*L |
|---|---|
| 20 mM 3PG | 10 |
| 20 m M NAD⁺ | 10 |
| PGDH | 10 |
| PSAT | 10 |
| 40 m M glutamate | 10 |
| 1 M HEPES (ₚH8.0) | 50 |
| H₂O | 900 |

**[Table 6]**

| | 3PGDH | PSAT |
|---|---|---|
| 1 | St | St |
| 2 | Tt | Tt |
| 3 | Tm | Tm |
| 4 | Tk | Tk |
| 5 | Te | Te |
| 6 | Tt | Tk |
| 7 | Tk | Tt |

| | | |
|---|---|---|
| St: Sulfolobus tokodaii Tt: Thermus thermophilus Tm: Thermotoga maritima Tk: Thermococcus kodakarensis Te: Thermosynechococcus elongates | | |

### (Investigation of L-cysteine production)

### [Material and technique]

### · Used enzyme gene, strain, and cultivation

A list of the enzyme genes used in this study is shown in Table 7. Fructose-1,6-bisphosphate aldolase derived from Thermus thermophilus HB8 (TtFBA), glyceraldehyde-3-phosphate dehydrogenase derived from Thermoproteus tenax (TteGAPN), glutamate dehydrogenase derived from Thermoproteus tenax (TteGDH), 3-phosphoglycerate dehydrogenase derived from Thermococcus kodakarensis KOD1 (TkPGDH), and phosphoserine aminotransferase derived from Thermococcus kodakarensis KOD1 (TkPSAT) ligated the gene obtained by PCR amplification of a genomic DNA of each of microorganisms to pET21a, and O-phosphoserine sulfhydrylase derived from Aeropyrum pernix (AmCysS), and NADH oxidase derived from Thermococcus profundus (TpNOX) ligated a synthetic DNA to pET21a, the expression was performed under T7 promoter control. All of these gene expression vectors were introduced into Rosetta 2 (DE3) pLysS manufactured by Novagen. Glucokinase derived from T. thermophilus HB8 (TtGK), glucose phosphate isomerase derived from T. thermophilus HB8 (TtGPI), phosphofructokinase derived from T. thermophilus HB8 (TtPFK), triose phosphate isomerase derived from T. thermophilus HB8 (TtTIM), and polyphosphate kinase derived from T. thermophilus HB27 (TtPPK) ligated a synthetic DNA to pRCI under lamnbda PR promoter control, and introduced to an E. coli DH5αstrain. The E. coli DH5αwas aerobically cultured at 37°C in a Luria-Bertani medium in which 100 mg/L of ampicillin had been added, and the Rosetta 2 (DE3) pLysS was aerobically cultured at 37°C in a Luria-Bertani medium in which 100 mg/L of ampicillin and 34 mg/L of chloramphenicol had been added. In the late logarithmic growth phase, 0.2 mM IPTG was added to the culture solution, or heat induction (42°C) was performed to induce a desired enzyme gene.

**[Table 7]**

| | Enzyme name | EC number | Derivation | Abbreviation | Expression vector (promoter) |
|---|---|---|---|---|---|
| 1 | Glucokinase | 2.7.1.2 | ***Thermus thermophilus*** HB8 | TtGK | pRC1 (lambda PR) |
| 2 | Glucose phosphate isomerase | 5.3.1.9 | *Thermus thermophilus* HB8 | TtGPI | pRC1 (lambda PR) |
| 3 | Phosphofructokinase | 2.7.1.1 | ***Thermus thermophilus*** HB8 | TtPFK | pRC1 (lambda PR) |
| 4 | Fructose-1,6-bisphosphate aldolase | 4.1.2.13 | *Thermus thermophilus* HB8 | TtFBA | pET21 a (T7) |
| 5 | Triose phosphate isomerase | 5.3.1.1 | ***Thermus** thermophilus* HB8 | TtTIM | ₚRC1 (lambda PR) |
| 6 | Glyceraldehyde-3-phosphate dehydrogenase | 1.2.1.90 | ***Thermoproteos tenax*** | TteGAPN | pET21 a (T7) |
| 7 | 3-phosphoglycerate dehydrogenase | 1.1.1.95 | ***Thermococcus kodakarensis*** KOD1 | TkPGDH | pET21 a (T7) |
| 8 | Phosphoserine aminotransferase | 2.6.1.52 | ***Thermococcus kodakarensis*** KOD1 | TkPSAT | pET21 a (T7) |
| 9 | O-phosphoserine sulfhydrylase | 2.5.1.65 | ***Acropyrum pernix*** | ApCysS | pET21a (T7) |
| 10 | Polyphosphate kinase | 2.7.4.1 | ***Thermus thermophilus*** HB27 | TtPPK | pET21 a (T7) |
| 11 | NADH oxidase | 1.6.3.4 | ***Thermococcus profundus*** | TpNOX | pET21 a (T7) |
| 12 | Glutamate dehydrogenase | 1.4.1.3 | ***Thermoproteus tenax*** | TteGDH | pET21 a (T7) |

### · Preparation of enzyme solution

The wet cells of recombinant E. coli were suspended in 50 mM HEPES-NaOH (pH 8.0) so as to be 200 mg wet cells/ml. The suspension was subjected to sonication to disrupt the bacterial cells, and a cell-free extract was obtained. The cell-free extract was heat-treated at 70°C for 30 minutes to denature the host-derived proteins. The supernatant in which cell residues and denatured proteins had been removed by centrifugation was used for activity measurement as a crude enzyme solution.

### · Enzymatic activity

For the activity measurement, 100 mM HEPES-NaOH (pH 8.0) was used, and all of the reactions were performed at 70°C. The TtGK was measured by the concentration of NADH generated by coupling the TtGK with a downstream pathway up to TteGAPN with the monitoring of the absorbance at 340 nm. TtGPI, TtPFK, TtFBA, TtTIM, and TteGAPN were also measured by a similar technique. The TkPGDH was measured by the concentration of NADH generated by coupling the TkPGDH with TkPSAT with the monitoring of the absorbance at 340 nm. By adding 20% trichloroacetic acid in an amount equal to the reaction solution into ApCysS, the resultant mixture denatured proteins, and after terminating the reaction, the concentration of L-cysteine was measured by performing a ninhydrin test of *4. As to TtPPK, the reaction was performed by coupling of GK and glucose-6-phosphate dehydrogenase (G6PDH) (manufactured by Thermostable Enzyme Laboratory Co., Ltd.). TpNOX was measured by the concentration of NADH decreasing due to the reaction with the monitoring of the absorbance at 340 nm. TteGDH was measured by the concentration of NADH accumulating due to the reaction with the monitoring of the absorbance at 340 nm. From these measurement results, the production amount of L-cysteine was measured by using glucose as a substrate with the addition of a required amount of a crude enzyme solution. Note that quantification of the L-cysteine was performed by a ninhydrin test. *4: GaitondeMK (1967) A spectrophotometric method for the direct determination of cysteine in the presence of other naturally occurring amino acids. Biochem J. 104(2): 627-633

### · Production test of L-cysteine

On the basis of the results of the measurement of the enzymatic activity, the productivity of L-cysteine was measured from glucose with the addition of a required amount of a crude enzyme solution. The measurement was performed in a reaction solution including 100 mM HEPES, 5 mM MgCl₂, 0.5 mM MnCl₂, 10 mM NAD⁺, 1 mM ATP, 1 mM G1P, 2.5 mM glucose, 5 mM glutamic acid, 5 mM sodium sulfide, 32 mM ammonium acetate, and 1 mM polyphosphoric acid (the average chain length: 60) as a reaction composition. The L-cysteine was quantitated by a ninhydrin test.

### · Results of production test of L-cysteine

The enzymes used in Examples were TtGK, TtGPI, TtPFK, TtFBA, TtTIM, TteGAPN, TkPGDH, TkPSAT, and ApCysS. The reaction composition was set to the composition under the condition of 1 of Table 8, and the reaction was performed at 70°C. Into a solution obtained by adding all of the enzymes described above into a reaction solution excluding glucose, glucose was added, and the reaction was initiated. The L-cysteine was quantitated in 15 minutes after the reaction initiation. As a result, 17.3 mg/L of L-cysteine was able to be confirmed.

### · Effect of addition of coenzyme regeneration system

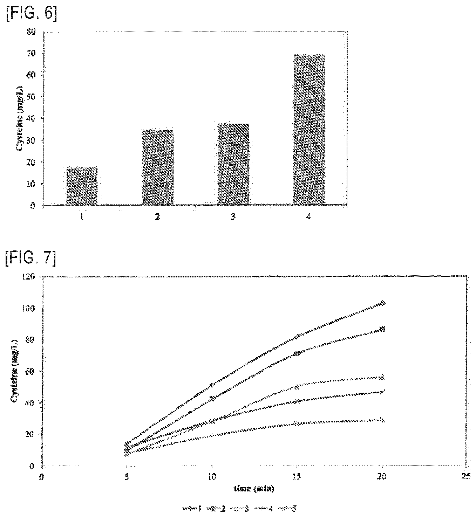

As a synthetic pathway of L-cysteine, NAD⁺ being a coenzyme was used. Investigation was performed for the purpose of improving the production rate of L-cysteine by providing GDH and NOX and reducing NADH. The reaction was performed at 70°C with the reaction composition of Table 8. The cysteine was quantitated in 15 minutes after reaction initiation. As a result, as shown in Fig. 6, it was revealed that the production amount was increased when TteGDH or TpNOX was added to the reaction solution, and the production amount was further increased when both of TteGDH and TpNOX were added to the reaction solution.

**[Table 8]**

| | 1 | 2 | 3 | 4 |
|---|---|---|---|---|
| 1 M HEPES (pH8.0) | 10 | | | |
| 1 M MgCl₂ | 0.5 | | | |
| 1 M MnCl2 | 0.5 | | | |
| 100 mM NAD⁺ | 10 | | | |
| 100 mM ATP | 1 | | | |
| 100 mM G1P | 1 | | | |
| 100 mM glucose | 2.5 | | | |
| 500 mM glutamats | 1 | | | |
| 100 mM Na₂S | 5 | | | |
| 320 mM OHₐCOONH₄ | 10 | | | |
| TtGK | 1 | | | |
| TtPGI | 1 | | | |
| TtPFK | 1 | | | |
| TtFBA | 1 | | | |
| TtTIM | 2 | | | |
| TteGAPN | 10 | | | |
| TkPGDH | 2 | | | |
| TkPSAT | 2 | | | |
| ApCysS | 2 | | | |
| TpNOX | 0 | 10 | 0 | 10 |
| TteGDH | 0 | 0 | 2 | 2 |
| H₂O | up to 100 *µ*I | | | |
| cysteine (mg/L) | 17.3 | 34.2 | 37.3 | 68.9 |

### · Time-dependent change

Time-dependent change of a production test of L-cysteine was taken. The enzymes used in a test at this time were TtGK, TtGPI, TtPFK, TtFBA, TtTIM, TteGAPN, TkPGDH, TkPSAT, ApCysS, TtPPK, TpNOX, and TteGDH. Into a solution obtained by adding all of the enzymes into a reaction solution excluding glucose, glucose was added, and the reaction was initiated. Sampling was performed in 5, 10, 15, and 20 minutes after the reaction initiation, and the L-cysteine was quantitated. The reaction composition is shown in Table 9, and the reaction was performed at 70°C. The results are shown in Fig. 7. According to the results, it was revealed that when TteGDH or TpNOX was added, the production rate was increased. On the other hand, a steady state was obtained in 15 minutes after reaction initiation in 3, 4, and 5 of Table 9. Further, the production amount was increased also in 2 of Table 9, however, the speed was gentle. On the other hand, the production was successively performed in 1 of Table 9, and the production rate was not decreased so much even as compared with the case in 2. Therefore, it was suggested that it was important to regenerate ADP to ATP by TtPPK for a long-time reaction.

**[Table 9]**

| | 1 | 2 | 3 | 4 | 5 |
|---|---|---|---|---|---|
| 1 M HEPES (pH8.0) | 40 | | | | |
| 1 M MgCl₂ | 2 | | | | |
| 1 M MnCl₂ | 2 | | | | |
| 100 mM NAD⁺ | 40 | | | | |
| 100 mM ATP | 4 | | | | |
| 100 mM G1P | 4 | | | | |
| 100 mM glucose | 10 | | | | |
| 500 mM glutamte | 4 | | | | |
| 100 mm Na₂S | 20 | | | | |
| 320 mM CH₃COONH₄ | 40 | | | | |
| TkGK | 4 | | | | |
| TkPGI | 4 | | | | |
| TkPFK | 4 | | | | |
| TkFBA | 4 | | | | |
| TkTIM | 8 | | | | |
| TteGAPN | 40 | | | | |
| TkPGDH | 8 | | | | |
| TkPSAT | 8 | | | | |
| TkCysS | 8 | | | | |
| TpNOX | 40 | 40 | 0 | 40 | 0 |
| TteGD H | 8 | 8 | 8 | 0 | 0 |
| TtPPK | 40 | 0 | 0 | 0 | 0 |
| PolyP | 40 | | | | |
| H₂O | up to 400 *µ*L | | | | |

From the above, in the present invention, by the combination with heat-resistant enzymes, L-cysteine can be produced by using a raw material that can be metabolized by the glycolytic pathway, for example, glucose or glycerol, or starch. In particular, O-phosphoserine efficiently produces a pathway synthesized from 3PG via HPV, and further by incorporating a step of resynthesizing a nicotinamide coenzyme that is a coenzyme, the production of L-cysteine can be performed for a long period of time.

## Claims

1. A method for producing O-phosphoserine, comprising acting phosphoserine aminotransferase (PSAT) and 3-phosphoglycerate dehydrogenase (PGDH) that are each derived from a thermophilic bacterium on 3-phosphoglyceric acid (3PG) at a pH of 6 to 8 to generate O-phosphoserine.

2. A method for producing L-cysteine, comprising the production step according to claim 1.

3. The method for producing L-cysteine according to claim 2, further comprising adding glutamate dehydrogenase or NADH oxidase to produce the L-cysteine.

## Patentansprüche

1. Verfahren zur Herstellung von O-Phosphoserin, umfassend das Wirkenlassen von Phosphoserin-Aminotransferase (PSAT) und 3-Phosphoglycerat-Dehydrogenase (PGDH), die jeweils von einem thermophilen Bakterium stammen, auf 3-Phosphoglycerinsäure (3PG) bei einem pH-Wert von 6 bis 8, um O-Phosphoserin zu erzeugen.

2. Verfahren zur Herstellung von L-Cystein, umfassend den Herstellungsschritt nach Anspruch 1.

3. Verfahren zur Herstellung von L-Cystein nach Anspruch 2, weiter umfassend das Zugeben von Glutamat-Dehydrogenase oder NADH-Oxidase, um das L-Cystein herzustellen.

## Revendications

1. Procédé de production d'O-phosphosérine, comprenant l'action de phosphosérine aminotransférase (PSAT) et de 3-phosphoglycérate déshydrogénase (PGDH) qui sont chacune dérivées d'une bactérie thermophile sur de l'acide 3-phosphoglycérique (3PG) à un pH de 6 à 8 pour générer de l'O-phosphosérine.

2. Procédé de production de L-cystéine, comprenant l'étape de production selon la revendication 1.

3. Procédé de production de L-cystéine selon la revendication 2, comprenant en outre l'ajout de glutamate déshydrogénase ou de NADH oxydase pour produire la L-cystéine.
